# EUROPEAN PATENT SPECIFICATION

(11) **EP 4 272 659 B1**
(45) Date of publication and mention of the grant of the patent: **21.05.2025**
(21) Application number: 22182144.0
(22) Date of filing: 30.06.2022
(51) Int. Cl.: A61B 17/128

(54) **SURGICAL CLIP APPLIER WITH LOCKING STRUTURE**
CHIRURGISCHE CLIP-ANBRINGVORRICHTUNG MIT VERRIEGELUNGSSTRUKTUR
APPLICATEUR D'AGRAFES CHIRURGICALES AVEC STRUCTURE DE VERROUILLAGE

(30) Priority: 06.05.2022 TW 111117234
(43) Date of publication of application: 08.11.2023
(73) Proprietor: Taiwan Surgical Corporation, Hsinchu County 302 (TW)
(72) Inventor: Lin, Wu Shen, 326 Taoyuan City (TW); Wang, Yun Hsiang, 406015 Taichung City (TW)
(74) Representative: Cabinet Chaillot

(56) References cited:
- AU-A1- 2013 254 887
- TW-B- I 690 295
- TW-B- I 698 217
- US-A1- 2004 097 970
- US-A1- 2014 316 441

## Description

### FIELD OF INVENTION

The present invention relates to a surgical clip applier, especially to a surgical clip applier with a locking structure.

### BACKGROUND OF THE INVENTION

In a surgical operation process, in order to avoid blood over losing from a surgical operation site, a surgical clip applier is usually applied to close a blood vessel by applying a clip on the blood vessel. The conventional way of using the surgical clip applier is locating the jaw which is mounted at a distal end of the surgical clip applier to a position near the target blood vessel by inserting the long rod-shaped surgical clip applier into the surgical operation site of the human body. Then, a handle at a proximal end of surgical clip applier is operated to make the jaw apply a clip on the blood vessel.

However, though the arranged clips can be delivered to the jaw sequentially, it cannot be clearly noticed that whether the last clip is run out when using the clip applier. With the careless attention, it is easy for the jaw to directly clamp the blood vessel and damage the vascular tissue by operate the surgical clip applier continuously after the last clip is discharged. It not only affects the operation process, but also causes unnecessary harm to the patient's body.

US20140316441A1 and US20040097970A1 discloses a conventional surgical clip applier with scissors structure. And AU2013254887A1 discloses a conventional surgical clip applier with blocking structure.

Therefore, it is an urgent program in the related field to develop a surgical clip applier with a locking function after the last clip is discharged.

### SUMMARY OF THE INVENTION

To overcome the aforementioned shortcomings of the prior surgical clip applier, the present invention provides a surgical clip applier according to claim 1. Optional features are defined in the dependent claims.

The present invention forms the locking structure as the wedge plate is blocked from the elastic leaf and prevent the jaw from closed by the wedge plate. With the revealed features, the surgical clip applier with the locking structure can overcome the shortage of the prior surgical clip applier which cannot be clearly noticed that the last clip is run out. Combining the design of the effect of preventing the reverse movement with the tooth-locking structure, the pressing handle can be locked and cannot be worked by the any other pression again. Specifically achieves an advantage that using a tactile-cue to notice the user that the clip in the surgical clip applier is run out, and prevents the misused of the surgical clip applier when preparing or using.

### BRIEF DESCRIPTION OF THE DRAWINGS

Fig. 1 is a side view of surgical clip applier in accordance with the present invention;
Fig. 2 is a cross sectional side view of the surgical clip applier in Fig. 1;
Fig. 3 is an enlarged cross sectional side view of a first operation of the surgical clip applier in Fig. 1;
Fig. 4 is an enlarged cross sectional side view of a second operation of the surgical clip applier in Fig, 1;
Fig. 5 is an enlarged cross sectional side view of a third operation of the surgical clip applier in Fig. 1;
Fig. 6 is an enlarged cross sectional side view of a fourth operation of the surgical clip applier in Fig. 1;
Fig. 7 is an enlarged side view of a handle of the surgical clip applier in Fig, 1;
Fig. 8 is an enlarged partial side view of the surgical clip applier in Fig. 1;
Fig. 9A is a first operational side view of the surgical clip applier in Fig. 8;
Fig. 9B is a second operational side view of the surgical clip applier in Fig. 8;
Fig. 9C is a third operational side view of the surgical clip applier in Fig. 8.

### DETAILED DESCRIPTION OF THE PREFERRED EMBODIMENTS

In order to make purposes, technical solutions, and advantages of the present invention to be clearer, the following content provides some preferred embodiments in accordance with the present invention.

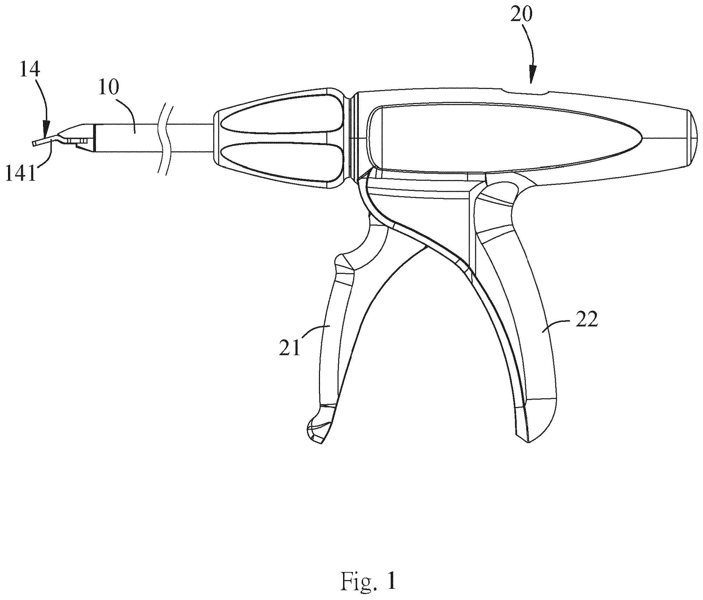

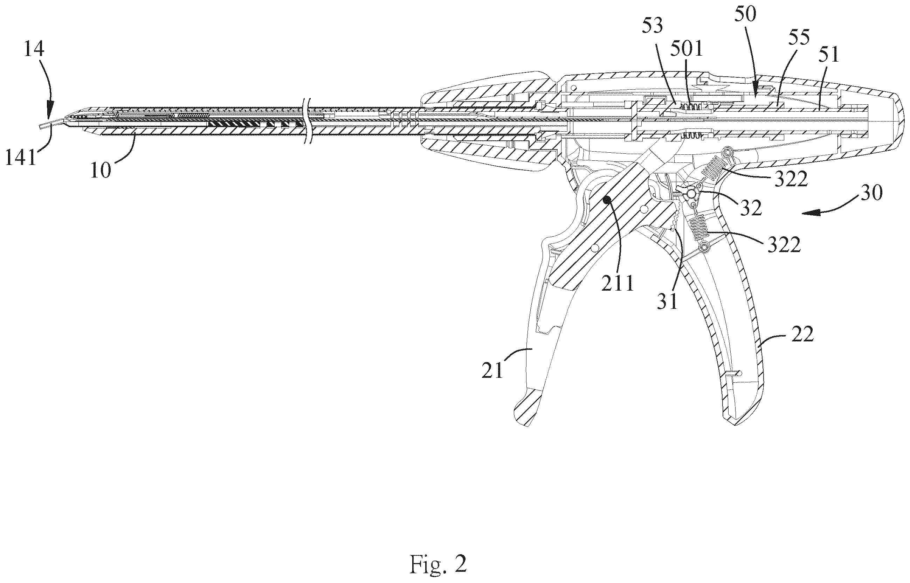

With reference to Fig. 1 to Fig. 3, a surgical clip applier in accordance with the present invention comprises a rod-shaped outer tube 10 extending along an axle, and a handle 20 mounted at a proximal end of the outer tube 10. A jaw 14 protrudes from the distal end of the outer tube 10 and has two jaw units 141 which are spaced apart from each other and are mounted respectively on opposite sides of the jaw 14. The space between two jaw units 141 defines a clamping space which can be narrowed or expanded according to the close or open movement from the two jaw units 141 driven by handle 20.

In the preferred embodiment, the proximal end of the jaw 14 comprises a fixing portion which is located in and connected to the bottom of the outer tube 10 fixedly. Two jaw units 141 are spaced apart from each other, protrude respectively toward the left and right sides of the fixing portion and outwardly from the distal end of the fixing portion, and extend out of the outer tube 10. The two jaw units 141 with the clamping space therebetween may exhibit an elastically deformed characteristic of closing to each other. The way of the two jaw units 141 driven by the handle 20 to close and to narrow the clamping space will be described in detail in the following paragraphs.

The outer tube 10 comprises a feeding rod 11, a clip carrier 12 and a wedge plate 13. The feeding rod 11, the clip carrier 12 and the wedge plate 13 extend along the axle and are arranged from a top to a bottom sequentially. The distal end of the feeding rod 11 forms a feeding unit 111 which protrudes downwardly. The proximal end of the feeding rod 11 is connected to the handle 20. The feeding rod 11 presents a reciprocating movement relative to the outer tube 10 according to the movement of the handle 20. When the feeding rod 11 is moved forwardly by the handle 20, the feeding unit 111 may extend down to the clip carrier 12 and push the distal most clip A into the clamping space.

The clip carrier 12 is placed in the outer tube 10 fixedly and is a square groove with an upward opening side. A storage space 121 is defined inside the clip carrier 12 and the storage space 121 can hold multiple clips A from a distal end to a proximal end in sequence inside. Near the distal end, a window 122 is defined through the bottom of the clip carrier 12 and forms a locking structure after the last clip A is run out. The forming method of the locking structure will be described in detail in the following paragraphs.

A block-like clip pusher 15 is located at the proximal side of the proximal most clip A and can be moved along the storage space 121. The clip pusher 15 can push the proximal most clip A and generates a pushing force to urge the multiple clips A to move toward to the distal end of outer tube 10. When the feeding rod 11 pushes the distal most clip A to the clamping space, the multiple remaining clips A can be pushed toward and supplemented to the distal end with the pushing force.

The manner of the clip pusher 15 to push the clips A is not limited in the present invention. A compressed-state spring can be placed at the proximal end of the clip pusher 15, so that the clip pusher 15 can provide the pressing force to the clips A continuously. Alternatively, the clip pusher 15 can also be moved to the distal end of outer tube 10 by driving the handle 20 and push the multiple clips A forwardly.

The clip pusher 15 comprises an elastic leaf 151 which extends toward the distal end of the outer tube 10 downwardly. When the clip pusher 15 is placed in the storage space 121, the elastic leaf 151 is elastically deformed and at least one part of the elastic leaf 151 abuts against the bottom surface of the clip carrier 12. The elastic leaf 151 moves to the distal end of the outer tube 10 sequentially while the clip pusher 15 pushes the multiple clips A.

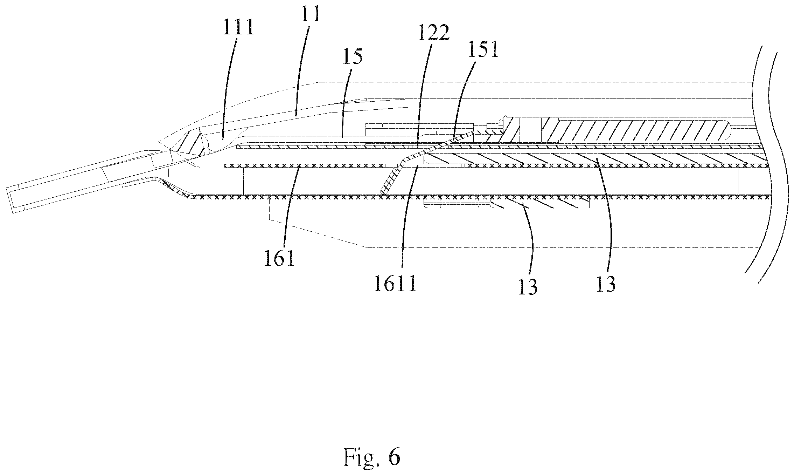

Referring to the Fig.6, when the handle 20 is triggered again after the last clip A is ejected, the clip pusher 15 drives the elastic leaf 151 to move to the position aligning with the window 122. At mean time, the elastic leaf 151 can extend toward the window 122 by its own elastic restoring force and extend through the bottom side of the clip carrier 12. Then, the wedge plate 13 which is located beneath the clip carrier 12 is blocked from its running direction by the elastic leaf 151. The manner of which the elastic leaf 151 blocks the wedge plate 13 will be described in detail in the following paragraphs.

The proximal end of the wedge plate 13 is connected to the handle 20. The wedge plate 13 presents a reciprocating movement relative to the outer tube 10 according to the movement of the handle 20. When the wedge plate 13 is moved distally, the distal end of the wedge plate 13 may urge the two jaw units 141 to close each other and narrow the clamping space. Preferably, the distal end of the wedge plate 13 covers around the left side and right side of the proximal end of the jaw 14. When the distal end of the wedge plate 13 is moved forward and aligned with the two jaw units 141 which protrude outwardly toward the left side and right side, the two jaw units 141 are urged by the wedge plate 13, the clamping space may be closed, and the clip A which is located in the clamping space may be compressed to be deformed.

Furthermore, a clip guard plate 16 is placed between the jaw 14 and the wedge plate 13. The clip guard plate 16 comprises a top guard section 161 located at an upside of the jaw 14, a bottom guard section 162 located at bottom side of the jaw 14, and a connected section connected between the top guard section 161 and the bottom guard section 162. A stop portion 163 is formed at the distal end of the bottom guard section 162, protrudes out from the outer tube 10 to a position corresponding to at least a part of the clamping space. The stop portion 163 can guide the clip A and prevent the clip A from falling off from the clamping space when the feeding rod 11 is pushing. Also, in order to avoid the poor position that causes a blood vessel unable to be effectively clamped by the clip A, the stop portion 163 can limit the depth of a blood vessel placed into the clamping space, and optimize the operation smoothness of the surgical clip applier.

In one of the preferred embodiments of the present invention, near the distal end of the window 122, a top guard window 1611 is defined through the top guard section 161. Thus, the elastic leaf 151 can sequentially extend through the window 122 and the top guard window 1611 when the elastic leaf 151 is moved toward the distal end and the elastic leaf 151 can block the wedge plate 13 after extending through the window 122 and the top guard window 1611.

In certain embodiment, a bottom guard window, near the distal end of the top guard window 1611 is defined through the bottom guard section 162. It is worthy of notice that a relative positional relationship between the bottom guard window and the top guard window 1611 is similar to the relative positional relationship between the top guard window 1611 and the window 122 so that the window 122, the top guard window 1611 and the bottom guard window are arranged from top to bottom and toward to the distal end in sequence. Thus, the elastic leaf 151 can sequentially extending through the window 122, the top guard window 1611 and the bottom guard window when the elastic leaf 151 is moved toward the distal end to strengthen the effect provided by the elastic leaf 151 to block the wedge plate 13.

Moreover, at least a proximal part of the top guard window 1611 is overlapped with at least a distal part of the window 122, and at least a proximal part of the bottom guard window is overlapped with the at least a distal part of the top guard window 1611. So that the elastic leaf 151 can extend through the window 122, the top guard window 1611 and the bottom guard window smoothly. Preferably, the feature of the bottom guard window is not necessary in this preferred embodiment, only the top guard window 1611 defined through the top guard section 161 also can improve the blocking effect of the locking structure.

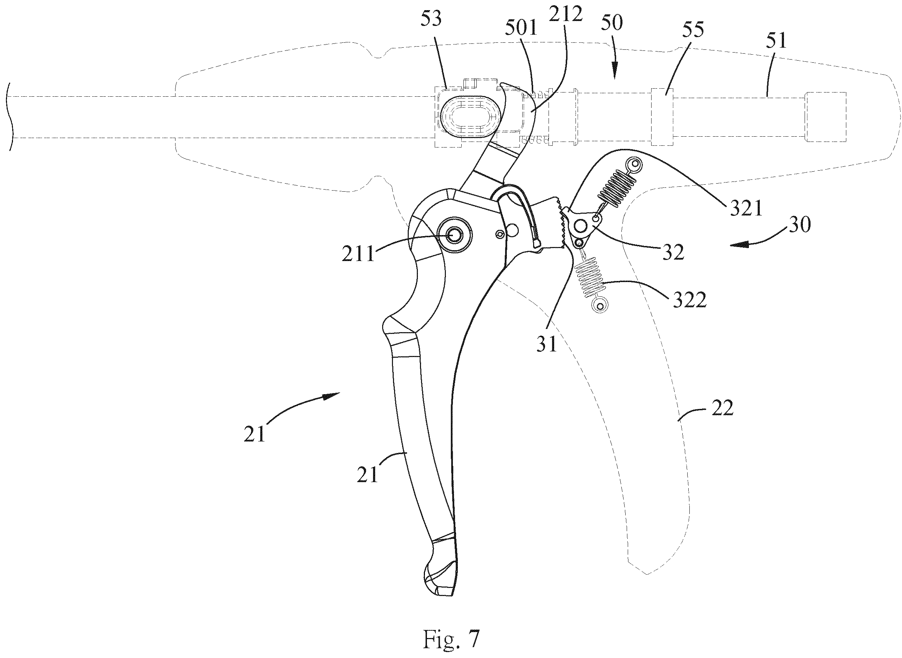

With reference to Fig. 7, the feature of the handle 20 is not limited in the present invention. The present embodiment applies the features disclosed in the previous documents TWI698217 and TWI703019. The handle 20 is a trigger structure including a pressing handle 21 and a fixed handle 22. The pressing handle 21 is a rod-like shape and forms a pivot portion 211 which can pivot relative to the fixed handle 22 at the top side. A hook section 212 extends upwardly from the pivot portion 211. When the pressing handle 21 is pivoted toward the fixed handle 22, the hook section 212 may drive the feeding rod 11 and the wedge plate 13 to move toward the distal end of outer tube 10 to make the clip A be fired when the handle 20 is triggered.

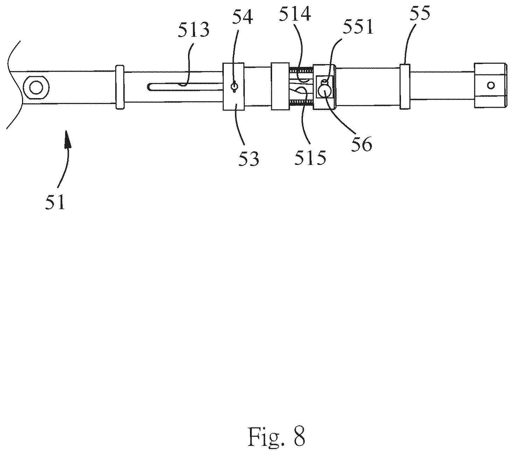

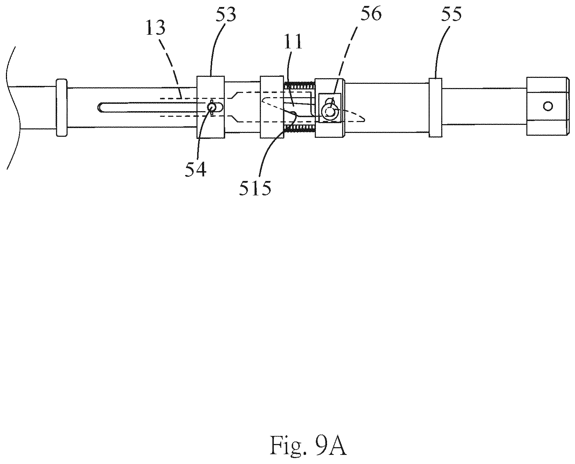

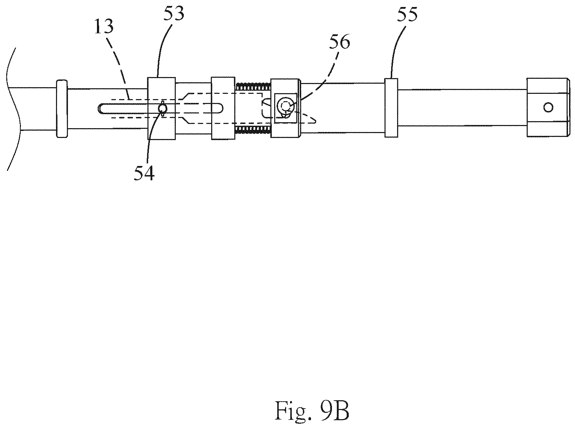

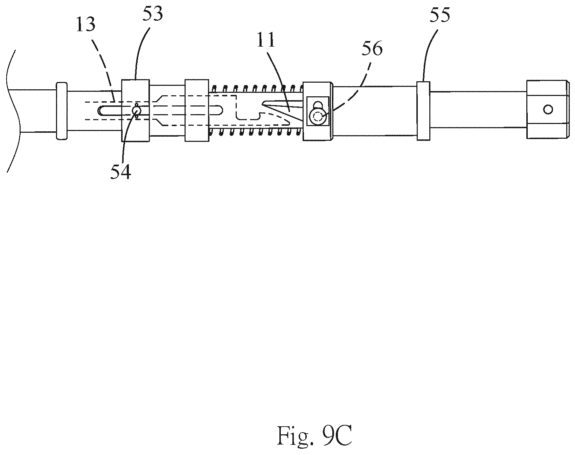

Referring to Fig. 8 and Fig. 9, the connection method of the feeding rod 11 and the wedge plate 13 with the handle 20 is not limited in the present invention. The present embodiment further referring to the features disclosed in the previous document TWI690295. The feeding rod 11 is a slice-rod shape with a proximal end extending though out the outer tube 10. A feeding rod long hole and a feeding rod hole are defined radially though the proximal end of the feeding rod 11. The feeding rod long hole is an elongated hole extending along a distal-proximal direction and located at the distal end of the feeding rod hole.

The wedge plate 13 is a slice-rod shape with a proximal end extending though out the outer tube 10. A wedge plate hole corresponding to the feeding rod long hole is defined radially though the wedge plate 13. The proximal end of the wedge plate 13 forms a hook extending in the proximal direction. The hook inwardly forms a notch from a side of the hook, and the feeding rod hole corresponds to the notch in position.

A drive element 50 is connected between the outer tube 10 and the handle 20 and comprises a slide bushing 51. A positioning long hole 513 corresponding to the feeding rod long hole is defined through the slide bushing 51. The positioning long hole 513 is an elongated hole extending in the distal-proximal direction. In correspondence with the position of the notch, an off-hooking long hole 514 is defined through the slide bushing 51, and the off-hooking long hole 514 is an elongated hole extending in the distal-proximal direction. An inclined plane 515 is formed at the distal end of the off-hooking long hole 514, and the inclined plane 515 is inclined toward the distal and the opening side of the notch.

A first bushing 53 is sheathed on the slide bushing 51 at a position where the feeding rod positioning pin long hole 513 is defined. The first bushing 53 is connected to the hook section 212 and can be moved toward the distal-proximal direction along the slide bushing 51. A first pin hole is defined through the first bushing 53. A first pin 54 is inserted through the first pin hole, the feeding rod long hole and the wedge plate hole sequentially. Thus, when the first bushing 53 is moved forward, the first pin 54 can drive the wedge plate 13 to move at the mean time.

A second bushing 55 is sheathed on the slide bushing 51 at a position in which the off-hooking long hole 514 is defined. The second bushing 55 is moved toward the distal-proximal direction along the slide bushing 51. A drive spring 501 is compressed and abuts against between the first bushing 53 and second bushing 55. A second pin hole 551 is defined through the second bushing 55 and has an extending direction same as the notch. An off-hooking pin 56 is inserted through the second pin hole 551, the off-hooking long hole 514 and the notch sequentially, and is fixed in the feeding rod hole at the proximal side of the feeding rod 11. The off-hooking pin 56 can drive the feeding rod 11 to move along the off-hooking long hole 514.

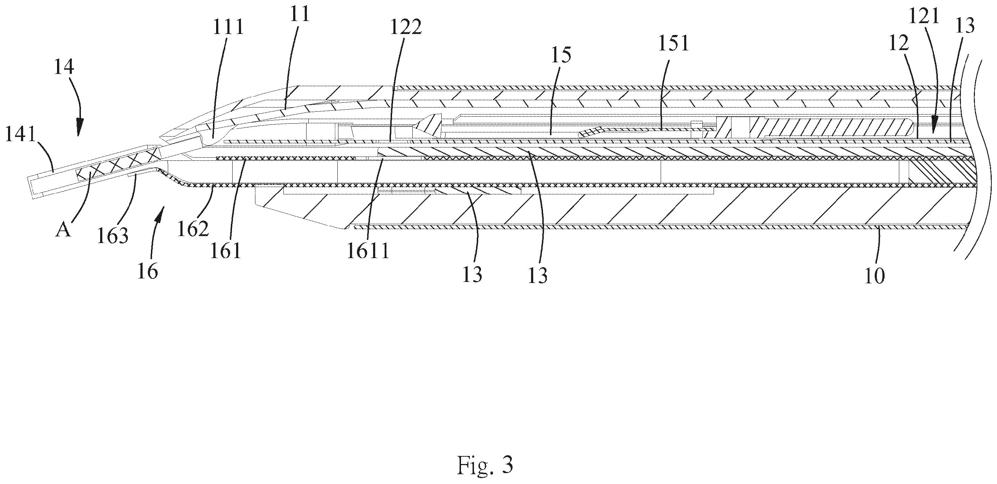

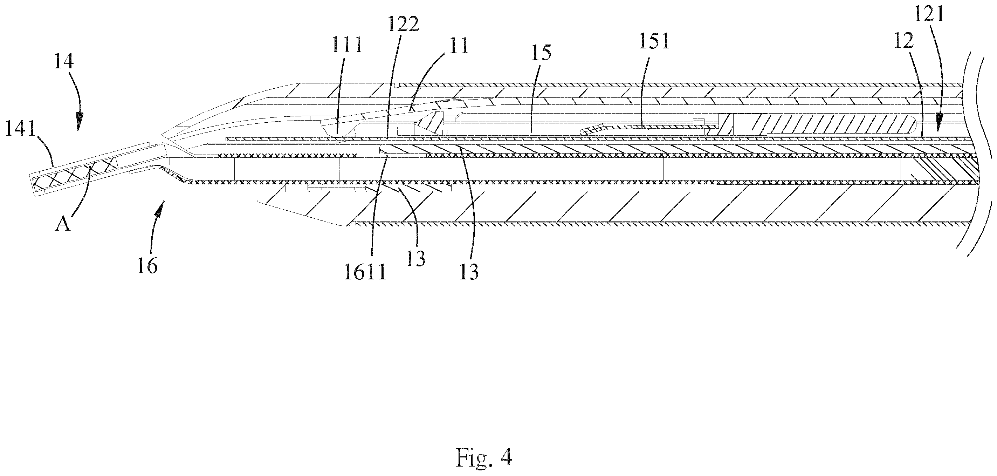

With reference to Fig. 3, Fig. 4 and Fig. 9A to Fig. 9C, since an initiated position, the off-hooking pin 56 abuts against an inner side of the notch, so that the hook at proximal side of the wedge plate 13 can hook the off-hooking pin 56. The drive spring 501 is maintained in a compressed statue.

When the handle 20 is triggered, the first slide bushing 51 drives the wedge plate 13 to move forward distally. Because the wedge plate 13 is hooking with the off-hooking pin 56, the second bushing 55 and the feeding rod 11 is moved forward altogether with the wedge plate 13. At the same time, the distal most clip A is moved to the clamping space by the feeding unit 111, and the clip pusher 15 pushes the proximal most clip A to urge the rest of clips A to move toward the distal end.

With the handle 20 being triggered continuously, the off-hooking pin 56 is moved to the inclined plane 515. The off-hooking pin 56 is moved along the inclined plane 515 which is toward the open side of the notch and separated from the hook. At this time, the second bushing 55 is moved backward proximally due to the drive spring 501 and drives the off-hooking pin 56 and the feeding rod 11 to move back to an original position. In the meantime, the distal most clip A is already placed in the clamping space, and the feeding rod 11 is returned to the initiated position.

Since the handle 20 is still triggered, the wedge plate 13 is moved froward continuously until the two jaw units 141 are urged by the distal end of the wedge plate 13 and the clip A is clamped in the clamping space to be deformed. When the handle 20 is released, the first bushing 53 may drive the wedge plate 13 to move backward. The first bushing 53 then compresses the drive spring 501 and makes the hook to return to the original position with hooking the off-hooking pin 56. Thus, the whole steps of discharging a clip A of the surgical clip applier is completed.

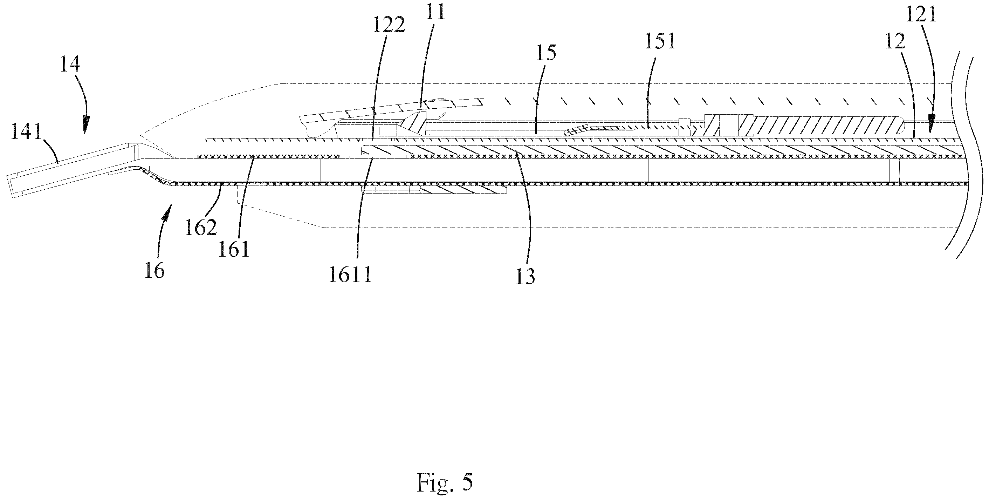

Referring to the Fig.5 to Fig.7, a preferred embodiment of the surgical clip applier with locking structure in accordance with the present invention is retriggered after the last clip A is discharged. With the last clip A is ejected, there are no more clips A in the clip carrier 12, and the clip pusher 15 is moved to the distal end of the clip carrier 12. When the pressing handle 21 is retriggered, the feeding rod 11 and the clip pusher 15 are moved forwardly and the clip pusher 15 drives the elastic leaf 151 to move to the position aligning with the window 122. As the distal moving of the clip pusher 15, the elastic leaf 151 extends through the window 122, the top guard window 1611 and the bottom guard window sequentially to make the wedge plate 13 be blocked in the running direction of the wedge plate 13.

Moreover, with the features disclosed in the previous documents TW 1703019, a tooth-locking structure 30 is located near the pressing handle 21 and comprises a ratchet 31 protruding from the pivot portion 211 and a toothed block 32. The toothed block 32 forms a toothed edge 321 facing the ratchet 31. When the handle 20 is triggered or released, the toothed edge 321 engages and moves along the ratchet 31 unidirectionally according to a pivot direction of the pressing handle 21. The toothed block 32 is pivoted toward the pivot direction at the same time. Two connect springs 322, at the other side of the toothed edge 321, are respectively settled at two opposite sides of the toothed block 32. After the toothed edge 321 moves unidirectionally along the ratchet 31, one of the connect springs 322 can provide a reverse pulling force to make the toothed block 32 move toward to a counter direction. The reverse pulling force generated by the two connect springs 322 can smooth the engagement of the toothed edge 321 and the ratchet 31 when the pressing handle 21 is pivoted for being triggered or released.

When the handle 20 is triggered again after the last clip A is ejected, since the wedge plate 13 is blocked and cannot move forward distally due to the elastic leaf 151. The toothed edge 321 is stuck in at least a part of the ratchet 31 and cannot be pivoted, so that the effect of preventing the reverse movement of the handle 20 is achieved.

The present invention forms the locking structure as the wedge plate 13 is blocked from the elastic leaf 151 and prevent the jaw from being closed by the wedge plate 13. With the revealed features, the surgical clip applier with the locking structure can overcome the shortage of the prior surgical clip applier which cannot be clearly noticed that the last clip is run out. Combining the design of the effect of preventing the reverse movement with the tooth-locking structure 30, the pressing handle 21 can be locked and cannot be worked by the any other pression again. Specifically achieves an advantage that using a tactile-cue to notice the user that the clip A in the surgical clip applier is run out, and prevents the misused of the surgical clip applier when preparing or using.

## Claims

1. A surgical clip applier comprising an outer tube (10) with a jaw (14) protruding from a distal end of the outer tube (10) and a handle (20) mounted at a proximal end of the outer tube (10), in the outer tube (10) from top to bottom sequentially arranged comprising:
a feeding rod (11) with a proximal end connected to the handle (20) presenting a reciprocate movement relative to the outer tube (10) from a movement of the handle (20), and the feeding rod (11) moving a distal most clip (A) into the jaw (14);
a clip carrier (12) which is a square groove with an upward opening side placed in the outer tube (10) fixedly and, the clip carrier (12) defining a storage space (121) inside and multiple clips (A) are held in the storage space (121) from a distal end to a proximal end in sequence, and a window (122) defined through a bottom of the distal end of the clip carrier (12); and
a clip pusher (15) located at a proximal side of a proximal most clip of the multiple clips (A) in the storage space (121) and the clip pusher (15) pushing the clips (A) to the distal end of the outer tube (10) from a proximal side of the clip carrier (12);
and a wedge plate (13) with a proximal end connected to the handle (20) presenting a reciprocate movement relative to the outer tube (10) from the movement of the handle (20), and the wedge plate (13) being applied to close the jaw (14);
**characterized in that** an elastic leaf (151) extends toward the distal end of the outer tube (10) from the clip pusher (15), the elastic leaf (151) deformed elastically by abutting against a bottom surface of the clip carrier (12), the elastic leaf (151) being moved to the distal end sequentially according to a movement of the clip pusher (15); and
wherein
when the handle (20) is triggered again after a last clip (A) is fired, the clip pusher (15) drives the elastic leaf (151) to move to a position aligning with the window (122), the elastic leaf (151) extends through the window (122) and blocks the wedge plate (13) from moving to the distal end of the outer tube (10).

2. The surgical clip applier as claimed in claim 1 further comprising a clip guard plate (16) placed under the wedge plate (13), a top guard window (1611) defined through the clip guard plate (16) near the distal end of the window (122), wherein when the clip pusher (15) drives the elastic leaf (151) to align with the window (122), the elastic leaf (151) sequentially extends through the window (122) and the top guard window (1611) according to a moving direction of the elastic leaf.

3. The surgical clip applier as claimed in claim 1, wherein
a proximal end of the jaw (14) comprises a fixing portion located in and connected with the outer tube (10);
a distal end of the wedge plate (13) covers around a left side and a right side of a proximal end of the jaw (14), and the jaw (14) is urged by the wedge plate (13) when the wedge plate (13) is moved toward to the distal end of the outer tube; and
the clip guard plate (16) comprises:
a top guard section (161) located at an upside of the jaw (14), and a top guard window (1611) defined through the top guard section (161); and
a bottom guard section (162) located at a bottom side of the jaw (14) and a bottom guard window defined through the bottom guard section (162), wherein the window (122), the top guard window (1611) and the bottom guard window are arranged in order from top to bottom and toward the distal end of the outer tube (10), and the elastic leaf (151) sequentially extends through the window (122), the top guard window (1611) and the bottom guard window when the clip pusher (15) moving toward the distal end the outer tube (10).

4. The surgical clip applier as claimed in claim 3, wherein at least a proximal part of the top guard window (1611) is overlapped with the at least a distal part of the window (122), and at least a proximal part of the bottom guard window is overlapped with the at least a distal part of the top guard window (1611).

5. The surgical clip applier as claimed in claim 3 further comprising a stop portion (163) formed at a distal end of the bottom guard section (162), and the stop portion (163) protruding out from the outer tube (10).

6. The surgical clip applier as claimed in claim 4, wherein the clip pusher (15) pushes the multiple clips (A) forwardly to the distal end of the outer tube (10) with being driven by the handle (20).

7. The surgical clip applier as claimed in claim 1 to 6, wherein the handle (20) is a trigger structure and comprises:
a fixed handle (22), formed at the proximal end of the outer tube (10); and
a pressing handle (21), a pivot portion (211) formed at a top side of the pressing handle (21), a hook (212) section extending upwardly from the pivot portion (211), wherein when the pressing handle (21) is pivoted toward the fixed handle (22), the hook section (212) drives the feeding rod (11) and the wedge plate (13) to the distal end of the outer tube (10) directly or indirectly.

8. The surgical clip applier as claimed in claim 7, wherein:
a tooth-locking structure (30) is located near the pressing handle (21) and comprises:
a ratchet (31) protruding from the pivot portion (211);
a toothed block (32) formed a toothed edge (321) facing the ratchet (31), when the handle (20) is triggered or released, the toothed edge (321) engages and moves along the ratchet (31) unidirectionally according to a pivot direction of the pressing handle (21); and
a connect springs (322) settled at the other side corresponding to the toothed edge (321) of the toothed block (32).

## Patentansprüche

1. - Wundklammerapplikator, umfassend ein äußeres Rohr (10) mit einer Klemmbacke (14), die von einem distalen Ende des äußeren Rohrs (10) hervorsteht, und einen Griff (20), der an einem proximalen Ende des äußeren Rohrs (10) montiert ist, wobei in das äußere Rohr (10) von oben nach unten aufeinanderfolgend Folgendes angeordnet ist:
ein Zufuhrstab (11) mit einem proximalen Ende, das mit dem Griff (20) verbunden ist, der durch eine Bewegung des Griffs (20) eine Hin- und Herbewegung in Bezug auf das äußere Rohr (10) aufweist, und wobei der Zufuhrstab (11) eine distalste Klammer (A) in die Klemmbacke (14) bewegt;
einen Klammerträger (12), der eine rechteckige Nut mit einer sich nach oben öffnenden Seite ist, die fest in dem äußeren Rohr (10) angeordnet ist, wobei der Klammerträger (12) einen Speicherraum (121) im Inneren definiert und mehrere Klammern (A) in dem Speicherraum (121) von einem distalen Ende zu einem proximalen Ende in Reihe gehalten werden, und ein Fenster (122), das durch einen Boden des distalen Endes des Klammerträgers (12) definiert ist; und
einen Klammerschieber (15), der sich auf einer proximalen Seite einer proximalsten Klammer der mehreren Klammern (A) in dem Speicherraum (121) angeordnet ist, und wobei der Klammerschieber (15) die Klammern (A) von einer proximalen Seite des Klammerträgers (12) zu dem distalen Ende des äußeren Rohrs (10) schiebt;
und eine Keilplatte (13), deren proximales Ende mit dem Griff (20) verbunden, die durch die Bewegung des Griffs (20) eine Hin- und Herbewegung in Bezug auf das äußere Rohr (10) aufweist, wobei die Keilplatte (13) zum Schließen der Backe (14) angewandt wird;
**dadurch gekennzeichnet, dass** sich ein elastisches Blatt (151) von dem Klammerschieber (15) in Richtung des distalen Endes des äußeren Rohrs (10) erstreckt, wobei das elastische Blatt (151) durch Anstoßen an eine Bodenfläche des Klammerträgers (12) elastisch verformt wird, wobei das elastische Blatt (151) gemäß einer Bewegung des Klammerschiebers (15) aufeinanderfolgend zu dem distalen Ende bewegt wird; und
wobei
wenn der Griff (20) erneut ausgelöst wird, nachdem eine letzte Klammer (A) abgefeuert wurde, der Klammerschieber (15) das elastische Blatt (151) antreibt, um sich zu einer Position zu bewegen, die mit dem Fenster (122) ausgerichtet ist, wobei sich das elastische Blatt (151) durch das Fenster (122) erstreckt und die Keilplatte (13) daran hindert, sich zu dem distalen Ende des äußeren Rohrs (10) zu bewegen.

2. - Wundklammerapplikator nach Anspruch 1, ferner umfassend eine Klammerschutzplatte (16), die unter der Keilplatte (13) platziert ist, ein oberes Schutzfenster (1611), das durch die Klammerschutzplatte (16) in der Nähe des distalen Endes des Fensters (122) definiert ist, wobei, wenn der Klammerschieber (15) das elastische Blatt (151) antreibt, um es mit dem Fenster (122) auszurichten, sich das elastische Blatt (151) gemäß einer Bewegungsrichtung des elastischen Blatts aufeinanderfolgend durch das Fenster (122) und das obere Schutzfenster (1611) erstreckt.

3. - Wundklammerapplikator nach Anspruch 1**,** wobei
ein proximales Ende der Klemmbacke (14) einen Befestigungsabschnitt umfasst, der sich in dem äußeren Rohr (10) befindet und damit verbunden ist;
ein distales Ende der Keilplatte (13) um eine linke Seite und eine rechte Seite eines proximalen Endes der Klemmbacke (14) herum abdeckt und die Klemmbacke (14) durch die Keilplatte (13) mitgenommen wird, wenn die Keilplatte (13) zu dem distalen Ende des äußeren Rohrs bewegt wird; und
die Klammerschutzplatte (16) Folgendes umfasst:
einen oberen Schutzabschnitt (161), der sich an einer Oberseite der Klemmbacke (14) befindet, und ein oberes Schutzfenster (1611), das durch den oberen Schutzabschnitt (161) definiert ist; und
einen unteren Schutzabschnitt (162), der an einer Unterseite der Klemmbacke (14) angeordnet ist, und ein unteres Schutzfenster, das durch den unteren Schutzabschnitt (162) definiert ist, wobei das Fenster (122), das obere Schutzfenster (1611) und das untere Schutzfenster in der Reihenfolge von oben nach unten und in Richtung des distalen Endes des äußeren Rohrs (10) angeordnet sind, und das elastische Blatt (151) sich aufeinanderfolgend durch das Fenster (122), das obere Schutzfenster (1611) und das untere Schutzfenster erstreckt, wenn sich der Klammerschieber (15) zu dem distalen Ende des äußeren Rohrs (10) bewegt.

4. - Wundklammerapplikator nach Anspruch 3, wobei mindestens ein proximaler Teil des oberen Schutzfensters (1611) mit dem mindestens einen distalen Teil des Fensters (122) überlappt, und mindestens ein proximaler Teil des unteren Schutzfensters mit dem mindestens einen distalen Teil des oberen Schutzfensters (1611) überlappt.

5. - Wundklammerapplikator nach Anspruch 3, ferner umfassend einen Anschlagabschnitt (163), der an einem distalen Ende des unteren Schutzabschnitts (162) gebildet ist, wobei der Anschlagabschnitt (163) aus dem äußeren Rohr (10) hervorsteht.

6. - Wundklammerapplikator Anspruch 4, wobei der Klammerschieber (15) die mehreren Klammern (A) mit Antrieb durch den Griff (20) nach vorne zu dem distalen Ende des äußeren Rohrs (10) schiebt.

7. - Wundklammerapplikator nach einem der Ansprüche 1 bis 6, wobei der Griff (20) eine Auslöserstruktur ist und Folgendes umfasst:
einen feststehenden Griff (22), der an dem proximalen Ende des äußeren Rohrs (10) gebildet ist; und
einen Druckgriff (21), einen Schwenkabschnitt (211), der an einer oberen Seite des Druckgriffs (21) gebildet ist, einen Hakenabschnitt (212), der sich von dem Schwenkabschnitt (211) nach oben erstreckt, wobei, wenn der Druckgriff (21) in Richtung des feststehenden Griffs (22) geschwenkt wird, der Hakenabschnitt (212) der Zufuhrstab (11) und die Keilplatte (13) direkt oder indirekt zu dem distalen Ende des äußeren Rohrs (10) antreibt.

8. - Wundklammerapplikator nach Anspruch 7, wobei:
sich eine Zahnverriegelungsstruktur (30) in der Nähe des Druckgriffs (21) befindet und Folgendes umfasst:
eine Sperrklinke (31), die aus dem Schwenkabschnitt (211) hervorsteht;
einen gezahnten Block (32), der eine gezahnte Kante (321) bildet, die der Sperrklinke (31) zugewandt ist, wobei die gezahnte Kante (321) in die Sperrklinke (31) eingreift und sich unidirektional gemäß einer Schwenkrichtung des Druckgriffs (21) entlang dieser bewegt, wenn der Griff (20) ausgelöst oder losgelassen wird; und
eine Verbindungsfeder (322), die auf der anderen Seite, die der gezahnten Kante (321) des Zahnblocks (32) entspricht, eingerichtet ist.

## Revendications

1. - Applicateur d'agrafes chirurgicales comprenant un tube extérieur (10) avec une mâchoire (14) faisant saillie à partir d'une extrémité distale du tube extérieur (10) et une poignée (20) montée à une extrémité proximale du tube extérieur (10), dans le tube extérieur (10) disposés en séquence de haut en bas se trouvent :
une tige d'alimentation (11) avec une extrémité proximale reliée à la poignée (20), présentant un mouvement de va-et-vient par rapport au tube extérieur (10) à la suite d'un mouvement de la poignée (20), et la tige d'alimentation (11) déplaçant jusque dans la mâchoire (14) une agrafe (A) la plus distale ;
un support d'agrafes (12) qui est une rainure carrée avec un côté d'ouverture vers le haut, placée de manière fixe dans le tube extérieur (10), et le support d'agrafes (12) définissant un espace de stockage (121) à l'intérieur et de multiples agrafes (A) étant maintenues dans l'espace de stockage (121) d'une extrémité distale à une extrémité proximale en séquence, et une fenêtre (122) définie à travers une partie inférieure de l'extrémité distale du support d'agrafes (12) ; et
un pousseur d'agrafes (15) situé à un côté proximal d'une agrafe la plus proximale parmi les multiples agrafes (A) dans l'espace de stockage (121), et le pousseur d'agrafes (15) poussant les agrafes (A) jusqu'à l'extrémité distale du tube extérieur (10) à partir d'un côté proximal du support d'agrafes (12) ; et
une plaque de calage (13) avec une extrémité proximale reliée à la poignée (20), présentant un mouvement de va-et-vient par rapport au tube extérieur (10) à la suite d'un mouvement de la poignée (20), et la plaque de calage (13) étant appliquée pour fermer la mâchoire (14) ;
**caractérisé par le fait qu'**une feuille élastique (151) s'étend vers l'extrémité distale du tube extérieur (10) à partir du pousseur d'agrafes (15), la feuille élastique (151) se déformant élastiquement en venant en butée contre une surface inférieure du support d'agrafes (12), la feuille élastique (151) étant déplacée en séquence jusqu'à l'extrémité distale en fonction d'un mouvement du pousseur d'agrafes (15) ; et
dans lequel lorsque la poignée (20) est à nouveau déclenchée après qu'une dernière agrafe (A) a été déclenchée, le pousseur d'agrafes (15) entraîne la feuille élastique (151) en déplacement jusqu'à une position alignée avec la fenêtre (122), la feuille élastique (151) s'étend à travers la fenêtre (122) et empêche la plaque de calage (13) de se déplacer jusqu'à l'extrémité distale du tube extérieur (10).

2. - Applicateur d'agrafes chirurgicales selon la revendication 1, comprenant en outre une plaque de protection d'agrafes (16) placée sous la plaque de calage (13), une fenêtre de protection supérieure (1611) définie à travers la plaque de protection d'agrafes (16) près de l'extrémité distale de la fenêtre (122), dans lequel, lorsque le pousseur d'agrafes (15) entraîne la feuille élastique (151) en alignement avec la fenêtre (122), la feuille élastique (151) s'étend en séquence à travers la fenêtre (122) et la fenêtre de protection supérieure (1611) en fonction d'une direction de déplacement de la feuille élastique.

3. - Applicateur d'agrafes chirurgicales selon la revendication 1, dans lequel
une extrémité proximale de la mâchoire (14) comprend une partie de fixation située dans et reliée au tube extérieur (10) ;
une extrémité distale de la plaque de calage (13) couvre un côté gauche et un côté droit d'une extrémité proximale de la mâchoire (14), et la mâchoire (14) est sollicitée par la plaque de calage (13) lorsque la plaque de calage (13) est déplacée vers l'extrémité distale du tube extérieur ; et
la plaque de protection d'agrafes (16) comprend :
une section de protection supérieure (161) située sur un côté supérieur de la mâchoire (14), et une fenêtre de protection supérieure (1611) définie à travers la section de protection supérieure (161) ; et
une section de protection inférieure (162) située sur un côté inférieur de la mâchoire (14) et une fenêtre de protection inférieure définie à travers la section de protection inférieure (162), la fenêtre (122), la fenêtre de protection supérieure (1611) et la fenêtre de protection inférieure étant disposées dans l'ordre de haut en bas et vers l'extrémité distale du tube extérieur (10), et la feuille élastique (151) s'étendant en séquence à travers la fenêtre (122), la fenêtre de protection supérieure (1611) et la fenêtre de protection inférieure lorsque le pousseur d'agrafes (15) se déplace vers l'extrémité distale du tube extérieur (10).

4. - Applicateur d'agrafes chirurgicales selon la revendication 3, dans lequel au moins une partie proximale de la fenêtre de protection supérieure (1611) est superposée à l'au moins une partie distale de la fenêtre (122), et au moins une partie proximale de la fenêtre de protection inférieure est superposée à l'au moins une partie distale de la fenêtre de protection supérieure (1611).

5. - Applicateur d'agrafes chirurgicales selon la revendication 3, comprenant en outre une partie d'arrêt (163) formée à une extrémité distale de la section de protection inférieure (162), et la partie d'arrêt (163) faisant saillie hors du tube extérieur (10).

6. - Applicateur d'agrafes chirurgicales selon la revendication 4, dans lequel le pousseur d'agrafes (15) pousse les multiples agrafes (A) vers l'avant jusqu'à l'extrémité distale du tube extérieur (10) sous l'action de la poignée (20).

7. - Applicateur d'agrafes chirurgicales selon les revendications 1 à 6, dans lequel la poignée (20) est une structure de déclenchement et comprend :
une poignée fixe (22), formée à l'extrémité proximale du tube extérieur (10) ; et
une poignée de pression (21), une partie pivot (211) formée sur un côté supérieur de la poignée de pression (21), une section crochet (212) s'étendant vers le haut à partir de la partie pivot (211), lorsque la poignée de pression (21) est pivotée vers la poignée fixe (22), la section crochet (212) entraînant la tige d'alimentation (11) et la plaque de calage (13) jusqu'à l'extrémité distale du tube extérieur (10), directement ou indirectement.

8. - Applicateur d'agrafes chirurgicales selon la revendication 7, dans lequel :
une structure de verrouillage de dent (30) est située à proximité de la poignée de pression (21) et comprend :
un rochet (31) faisant saillie à partir de la partie pivot (211) ;
un bloc denté (32) formé d'un bord denté (321) faisant face au rochet (31), lorsque la poignée (20) est déclenchée ou relâchée, le bord denté (321) s'engageant et se déplaçant le long du rochet (31) de manière unidirectionnelle selon une direction de pivot de la poignée de pression (21) ; et
un ressort de liaison (322) installé de l'autre côté correspondant au bord denté (321) du bloc denté (32).
